# EUROPÄISCHE PATENTSCHRIFT

(11) **EP 3 634 715 B1**
(45) Veröffentlichungstag und Bekanntmachung des Hinweises auf die Patenterteilung: **16.02.2022**
(21) Anmeldenummer: 18729111.7
(22) Anmeldetag: 05.06.2018
(51) Int. Cl.: B29C 49/46, B29C 49/06, B29C 49/42, B29L 31/00

(54) **VORRICHTUNG UND VERFAHREN ZUM BEHANDELN UND INSBESONDERE TRANSPORTIEREN VON KUNSTSTOFFVORFORMLINGEN**
DEVICE AND METHOD FOR HANDLING AND MORE PARTICULARLY TRANSPORTING PLASTIC PREFORMS
DISPOSITIF ET PROCÉDÉ POUR TRAITER ET EN PARTICULIER TRANSPORTER DES PRÉFORMES EN MATIÈRE PLASTIQUE

(30) Priorität: 06.06.2017 DE 102017112455
(43) Veröffentlichungstag der Anmeldung: 15.04.2020
(73) Patentinhaber: Krones AG, 93073 Neutraubling (DE)
(72) Erfinder: NEBL, Florian, 93073 Neutraubling (DE); WÜNSCHE, Dieter, 93073 Neutraubling (DE); SÖLLNER, Jürgen, 93073 Neutraubling (DE)
(74) Vertreter: Bittner, Bernhard
(86) Internationale Anmeldenummer: PCT/EP2018/064704
(87) Internationale Veröffentlichungsnummer: WO 2018/224472

(56) Entgegenhaltungen:
- EP-A1- 2 191 953
- EP-A1- 2 711 158
- EP-B1- 2 711 158
- US-A1- 2011 061 343
- US-A1- 2013 061 557
- US-A1- 2014 322 074
- US-A1- 2015 225 098

## Beschreibung

Die vorliegende Erfindung bezieht sich auf eine Vorrichtung und ein Verfahren zum Behandeln von Kunststoffvorformlingen und insbesondere zum Transportieren von Kunststoffvorformlingen. Derartige Vorrichtungen und Verfahren sind aus dem Stand der Technik seit Langem bekannt. Die Dokumente US2013/061557A1, US2011/061343A1 und EP2711158A1 beschreiben Vorrichtungen und Verfahren zum Behandeln von Kunststoffvorformlingen gemäß dem Stand der Technik. Dabei ist es insbesondere bekannt, dass Kunststoffvorformlinge ausgehend von einem Vorrat, wie etwa einer Preformschütte oder dergleichen, mittels verschiedenen Transportmitteln zu einer Blasformmaschine bzw. einem Ofen transportiert werden.

Im aktuellen Stand der Technik werden die Kunststoffvorformlinge in einer Zuführung mittels eines Silos, mittels Zuführbändern und einem Rollensortierer transportiert. Diese genannten Transport- und Sortierelemente können mit einfachen Abdeckungen gegen groben Schmutz aus der Umgebung abgeschottet werden. Gleiches gilt auch für eine im Stand der Technik oftmals vorhandene schräge Zuführschiene zwischen einem Rollensortierer und einer Blasmaschine. In der aus dem Stand der Technik bekannten Ausführung erfolgt in diesem Handlingsbereich der Kunststoffvorformlinge zwischen der Entnahme der Kunststoffvorformlinge aus einem Behälter bis hin zu einer Blasformmaschine eine hohe Zunahme einer Verkeimung. Diese Keime müssen in den nachfolgenden Verarbeitungsprozessen mit vergleichsweise hohem Aufwand wieder beseitigt werden bzw. die Vorformlinge sterilisiert werden.

Der vorliegenden Erfindung liegt daher die Aufgabe zugrunde, eine Verkeimung der Kunststoffvorformlinge auf dem Weg hin in Richtung einer Blasmaschine gering zu halten und/oder den Sterilisationsaufwand zu senken.

Diese Aufgabe wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Behandeln von Kunststoffvorformlingen und insbesondere zum Transportieren von Kunststoffvorformlingen weist eine Aufbewahrungseinrichtung auf, welche zum Aufbewahren einer Vielzahl von Kunststoffvorformlingen dient, sowie wenigstens eine Transporteinrichtung, welche die Kunststoffvorformlinge entlang eines vorgegebenen Transportpfads von der Aufbewahrungseinrichtung zu einer Verarbeitungseinrichtung transportiert. Bevorzugt sind wenigstens zwei, bevorzugt wenigstens drei Transporteinrichtungen vorgesehen, welche die Kunststoffvorformlinge transportieren.

Erfindungsgemäß ist wenigstens ein Abschnitt der Aufbewahrungseinrichtung und des Transportpfads von einer Gehäuseeinrichtung eingehaust und es ist eine Beaufschlagungseinrichtung vorgesehen, welche einen Innenraum dieser Gehäuseeinrichtung mit einem fließfähigen und insbesondere gasförmigen Medium beaufschlagt.

Mit anderen Worten ist bevorzugt auch wenigstens ein Abschnitt der Transporteinrichtung eingehaust. Bevorzugt kann es sich bei der Aufbewahrungseinrichtung um ein Silo handeln, bzw. einen Behälter, in dem eine Vielzahl von Kunststoffvorformlingen aufgenommen werden kann.

Unter einer Einhausung wird im Folgenden zumindest ein Umgeben des Transportpfads in dessen Umfangsrichtung verstanden, gegebenenfalls aber auch eine vollständige Einhausung, das heißt ein im Wesentlichen abgeschlossener Raum. Bevorzugt kann jedoch diese Einhausung auch Öffnungen aufweisen, um beispielsweise eine Be- und Entlüftung vorzunehmen und/oder um eine gewisse Strömung des gasförmigen Mediums innerhalb des Gehäuses bzw. der Einhausung zu erreichen. Insbesondere wird mittels dieser Öffnungen ein Luftstrom erzeugt.

Bevorzugt ist die Verarbeitungseinrichtung aus einer Gruppe von Verarbeitungseinrichtungen ausgewählt, welche Vorrichtungen zum Umformen von Kunststoffvorformlingen zu Kunststoffbehältnissen, insbesondere Blasformmaschinen, Sterilisationseinrichtungen zum Sterilisieren von Kunststoffvorformlingen, Öfen zum Erwärmen von Kunststoffvorformlingen und dergleichen enthält. Insbesondere handelt es sich bei der Verarbeitungseinrichtung um einen Ofen, der dazu geeignet und bestimmt ist, Kunststoffvorformlinge zu erwärmen.

Bei einer weiteren vorteilhaften Ausführungsform ist die gesamte Aufbewahrungseinrichtung und/oder der gesamte Transportpfad von einer oder mehreren Gehäuseeinrichtungen eingehaust. Bevorzugt sind sowohl die gesamte Aufbewahrungseinrichtung als auch der gesamte Transportpfad von einer Gehäuseeinrichtung eingehaust. Diese mehreren Gehäuseeinrichtungen können dabei derart miteinander verbunden sein, dass die Kunststoffvorformlinge von einer Gehäuseeinrichtung in eine weitere Gehäuseeinrichtung transportiertbar sind. Auch können benachbarte Gehäuseeinrichtungen miteinander gemeinsame Wandungen aufweisen.

Auf diese Weise wird eine kontrollierte Belüftung der Preformzuführung bereitgestellt, welche den gesamten Handlingsbereich der Kunststoffvorformlinge überdeckt, beispielsweise einen Kipper, ein Silo, Zuführbänder und auch Rollensortierer einschließlich Zuführschienen. Dabei wird dieser Bereich bevorzugt mit kontrollierter sauberer und gefilterter Luft versorgt. Auf diese Weise ist es möglich, in einer im Wesentlichen abgeschlossenen Umgebung einen beispielsweise 50- bis 100-fachen Luftwechsel pro Stunde zu gewährleisten. Durch diesen Luftwechsel und den dadurch entstehenden geringen Überdruck innerhalb der Einhausungen wird bevorzugt verhindert, dass verschmutzte Luft und/oder mit Keimen belastete Luft in die abgedeckten Bereiche eindringen kann und so wird eine unnötige Verkeimung der Kunststoffvorformlinge verhindert.

Es wird jedoch darauf hingewiesen, dass diese Vorgehensweise keine Sterilisation oder Reinraumhaltung ermöglicht, sondern wie oben erwähnt eine Beaufschlagung der Transportbereiche der Kunststoffvorformlinge mit relativ sauberer bzw. gefilterter Luft. Es wird jedoch eine Möglichkeit geschaffen, um den Aufwand für eine nachfolgende Sterilisierung zu verringern. Bevorzugt weisen dabei die Einhausungen bzw. Gehäuse auch Öffnungen auf, welche einen Luftstrom im Inneren der Einhausungen ermöglichen. Auf diese Weise können die Bereiche, in denen die Kunststoffvorformlinge transportiert werden, stets mit frischer und/oder gefilterter Luft versorgt werden.

Bevorzugt ist wenigstens eines der genannten Gehäuse stationär ausgebildet. Besonders bevorzugt sind mehrere der Gehäuse stationär ausgebildet und besonders bevorzugt sind sämtliche der genannten Gehäuse stationär ausgebildet. Dabei können die Gehäuse auch Öffnungen, wie insbesondere aber nicht ausschließlich Revisionsöffnungen aufweisen, welche einen Zugang zu den einzelnen Vorrichtungen wie etwa der Sortiereinrichtung ermöglichen, etwa zu Reparatur- und/oder Wartungszwecken.

Auf diese Weise wird eine Verkeimung und/oder Verschmutzung der Kunststoffvorformlinge in den Kunststoffvorformlingszuführungen verringert. Dies führt damit zu einer Aufwandsverringerung bei der Sterilisation in Folgeprozessen bzw. zur Erhöhung deren Effektivität bei einem gleichbleibenden Aufwand. Durch ein entsprechend gut ausgestaltetes Belüftungskonzept wird es weiterhin möglich, die Kunststoffvorformlingszuführung vor eventuell erforderlichen Reinräumen zu installieren oder auch eine Möglichkeit zu schaffen, bei der Reinräume komplett entfallen können.

Bei einer weiteren vorteilhaften Ausführungsform weist die Vorrichtung entlang des Transportpfads der Kunststoffvorformlinge eine Sortiereinrichtung und/oder Ausrichteinrichtung auf, welche dazu geeignet und bestimmt ist, die Kunststoffvorformlinge zu sortieren und/oder auszurichten. Aus dem Stand der Technik sind unterschiedliche Sortiereinrichtungen bekannt, wie beispielsweise Scheibensortierer oder Rollensortierer. Besonders bevorzugt weist die Sortiereinrichtung wiederum zwei voneinander beabstandete drehbare Rollen auf, zwischen denen die Kunststoffvorformlinge transportierbar sind.

Bevorzugt wird also ein sogenannter Rollensortierer vorgeschlagen. Bevorzugt sind dabei diese beiden drehbaren Rollen derart beabstandet, dass ein Grundkörper der Kunststoffvorformlinge durch einen zwischen den Rollen gebildeten Spalt treten kann, ein Tragring der Kunststoffvorformlinge jedoch von den Rollen abgestützt wird. Bei einer entsprechenden Drehung der Rollen ist dann so eine Sortierung der Kunststoffvorformlinge möglich.

Bevorzugt sind diese Transportrollen leicht geneigt, sodass die Kunststoffvorformlinge auch bei einer Drehung der Rollen in eine bestimmte Transportrichtung transportiert werden. An diese Sortiereinrichtungen können sich weitere Transporteinrichtungen anschließen, welche die Kunststoffvorformlinge bereits in einer vorbestimmten Ausrichtung, beispielsweise mit deren Tragringen bzw. Mündungen nach oben, transportieren.

Bei einer weiteren vorteilhaften Ausführungsform weist die Transporteinrichtung in ihrer Gesamtheit auch wenigstens einen sogenannten Steilförderer auf, der die Kunststoffvorformlinge nicht in einer horizontalen oder nicht nur in einer horizontalen Richtung fördert, sondern auch in einer vertikalen Richtung, insbesondere um Höhenunterschiede zu überbrücken. Insbesondere ist dieser Steilförderer dazu geeignet und bestimmt, die Kunststoffvorformlinge aufwärts zu fördern.

Bei einer weiteren bevorzugten Ausführungsform weist die Vorrichtung auch Ablaufschienen auf. Daneben kann die Vorrichtung auch wenigstens eine Inspektionseinrichtung aufweisen, welche dazu geeignet und bestimmt ist, bestimmte Bereiche der Kunststoffvorformlinge, wie etwa Mündungsbereiche oder auch Bereiche deren Grundkörper, zu inspizieren. Vorteilhaft sind dabei auch die Transportpfade der Kunststoffvorformlinge durch diese Inspektionseinrichtung eingehaust und/oder mit gefilterter Luft und/oder Sterilluft beaufschlagt.

Bei einer weiteren vorteilhaften Ausführungsform weist die Vorrichtung eine Filtereinrichtung zum Filtern des dem Innenraum der Gehäuseeinrichtung zuzuführenden gasförmigen Mediums auf. Insbesondere ist diese Filtereinrichtung dazu geeignet und bestimmt, Keime aus der Luft herauszufiltern.

Bevorzugt weist die Beaufschlagungseinrichtung eine Lüftereinrichtung zum Erzeugen eines Luftstroms auf und insbesondere eines Luftstroms innerhalb des Gehäuses. Vorteilhaft erstreckt sich dieser Luftstrom wenigstens zeitweise auch in der Transportrichtung der Kunststoffvorformlinge oder entgegen dieser Transportrichtung.

Vorteilhaft weist diese Lüftereinrichtung wenigstens zwei Lüftereinheiten auf. Bei einer weiteren vorteilhaften Ausführungsform weist wenigstens eine Lüftereinrichtung eine Leistung auf, welche zwischen 1.000 und 3.500 m³/h, bevorzugt zwischen 1.200 und 3.000 m³/h und bevorzugt zwischen 1.400 und 2.600 m³/h liegt. Generell ist die Leistung der Lüfter und/oder sind die genannten Volumina abhängig vom Layout der Anlage. Es wäre auch denkbar, dass nur eine Lüftereinrichtung vorgesehen ist.

Bei einer weiteren vorteilhaften Ausführungsform ist wenigstens eine Lüftereinrichtung innerhalb einer Gehäuseeinrichtung angeordnet, welche die Aufbewahrungseinrichtung wenigstens teilweise umgibt. So ist es beispielsweise möglich, dass die Lüftereinrichtungen in eine Silo- bzw. Kippereinhausung bzw. in der Einhausung der Aufbewahrungseinrichtung untergebracht sind. Dabei ist es beispielsweise möglich, dass die Lüftereinrichtung direkt oberhalb bzw. über einem Silo angebracht wird, oder auch seitlich, beispielsweise an der Wandung der Einhausung der Aufbewahrungseinrichtung. Daneben wäre es auch denkbar, dass die Lüftereinrichtung außerhalb des Gehäuses angeordnet ist.

Bei einer bevorzugten Ausführungsform weist ein Gehäuse, welches die Aufbewahrungseinrichtung umgibt, eine Öffnung auf, über welche Kunststoffvorformlinge zugeführt werden können. Dabei ist es beispielsweise möglich, dass in das Gehäuse Behälter mit Kunststoffvorformlingen beispielsweise über einen Palettentransport oder Stapler eingebracht werden. Die Öffnung selbst kann verschließbar sein, beispielsweise durch ein Roll- oder Schiebetor.

Bei einer weiteren vorteilhaften Ausführungsform weist die Beaufschlagungseinrichtung eine Versorgungsleitung auf, welche dazu geeignet und bestimmt ist, einen von einer Lüftereinrichtung erzeugten Gasstrom zu mehreren Positionen entlang des Transportpfads der Kunststoffvorformlinge zu verbringen. Dabei wird darauf hingewiesen, dass sich der Transportpfad der Kunststoffvorformlinge teilweise über größere Wegstrecken erstreckt. Auf diese Weise wird ein Versorgungsleitungssystem vorgeschlagen, welches dazu geeignet und bestimmt ist, einen Gasstrom in mehreren Bereichen der Vorrichtung zu erzeugen und insbesondere auch an mehreren Bereichen entlang des Transportpfads der Kunststoffvorformlinge.

Bei einer weiteren vorteilhaften Ausführungsform weist diese Versorgungsleitung eine Hauptleitung auf, welche bevorzugt eine oder mehrere Abzweigleitungen aufweist bzw. welche in eine diese Abzweigleitungen mündet. Dabei können diese Abzweigleitungen einzelnen Gehäuseteilen zugeführt werden. So können einzelne Gehäuseteile jeweils mit Luft versorgt werden.

Es wäre dabei möglich, dass die Vorrichtung ein vollständiges Gehäuse aufweist, welches den gesamten Transportpfad umgibt. Es wäre jedoch auch möglich, dass eine Vielzahl von Gehäusen vorgesehen ist, welche dazu geeignet und bestimmt sind, einen Transportpfad der Kunststoffvorformlinge kanalartig zu umgehen. Auf diese Weise kann das Volumen, welches mit dem Luftstrom beaufschlagt wird, relativ gering gehalten werden.

Bei einer weiteren vorteilhaften Ausführungsform weisen diese Versorgungsleitungen Sperreinrichtungen auf, welche dazu geeignet und bestimmt sind, Luftstrom zu drosseln. So ist es möglich, dass der Luftstrom über eine große Versorgungsleitung und über kleinere Stichleitungen in einen Einwurfbereich der einzelnen Transporteure geleitet wird. Dabei sind bevorzugt diese Stichleitungen mit Absperrschiebern ausgestattet, um den Luftstrom partiell in einzelnen Bereichen des Transportpfads zu steuern und insbesondere zu drosseln.

So ist es beispielsweise möglich, dass der Luftstrom über kleine Versorgungsleitungen in einen Einwurfbereich eines Rollensortierers, eines Rückführbandes und bevorzugt auch in einem Anfangsbereich von Ablaufschienen geleitet wird. Die Luft strömt bevorzugt mit der Förderrichtung zu dem jeweiligen Transportende und entweicht dort. Zu diesem Zweck können die einzelnen Gehäuseteile jeweils Auslassöffnungen vorsehen, welche es ermöglichen, dass ein gezielter Luftstrom geschaffen wird.

Bevorzugt sind wie erwähnt diese Versorgungsleitungen absperrbar bzw. der durch diese Versorgungsleitungen und insbesondere die oben erwähnten Abzweigleitungen strömende Luftstrom ist regulierbar und insbesondere drosselbar. So ist es möglich, dass die oben erwähnten Abzweigleitungen mit Absperrschiebern ausgestattet sind, um den Luftstrom zu steuern oder zu drosseln. So ist es möglich, die Luftströmungen innerhalb der einzelnen Einrichtungen individuell zu steuern und/oder zu regeln. Auch ist es denkbar und bevorzugt, dass die Leistung der wenigstens einen Lüftereinrichtung und allgemein die Leistungen der mehreren Lüftereinrichtungen regelbar sind.

Auch ist es beispielsweise möglich, dass sich an einen Rollensortierer eine weitere Transporteinrichtung wie eine Ablaufschiene anschließt.

Bei einer weiteren vorteilhaften Ausführungsform weist die Vorrichtung eine Erwärmungseinrichtung auf, welche dazu geeignet und bestimmt ist, die Kunststoffvorformlinge zu erwärmen. Diese Erwärmungseinrichtung ist bevorzugt in einer Transportrichtung der Kunststoffvorformlinge nach wenigstens einem Teil der oben erwähnten Transporteinrichtungen angeordnet. Bevorzugt ist zwischen der Aufbewahrungseinrichtung und der erwähnten Erwärmungseinrichtung keine Einrichtung zum Sterilisieren von Kunststoffvorformlingen vorgesehen. Im Stand der Technik ist es teilweise üblich, im Bereich des Transportpfads von der Aufbewahrungseinrichtung und der Erwärmungseinrichtung eine Sterilisationseinrichtung vorzusehen, welche dazu geeignet und bestimmt ist, Kunststoffvorformlinge zu sterilisieren. Durch die erfindungsgemäße Ausgestaltung der Vorrichtung kann auf eine derartige Sterilisierungseinrichtung verzichtet werden.

Die vorliegende Erfindung ist weiterhin auf ein Verfahren zum Behandeln von Kunststoffvorformlingen gerichtet, wobei in einer Aufbewahrungseinrichtung eine Vielzahl von Kunststoffvorformlingen aufbewahrt wird und diese Kunststoffvorformlinge mit wenigstens einer Transporteinrichtung entlang eines vorgegebenen Transportpfads von der Aufbewahrungseinrichtung zu einer Verarbeitungseinrichtung transportiert werden.

Erfindungsgemäß ist wenigstens ein Abschnitt der Aufbewahrungseinrichtung und des Transportpfads der Kunststoffvorformlinge von einer Gehäuseeinrichtung eingehaust und eine Beaufschlagungseinrichtung beaufschlagt einen Innenraum dieser Gehäuseeinrichtung mit einem fließfähigen und insbesondere gasförmigen Medium. Besonders bevorzugt handelt es sich bei diesem gasförmigen Medium um Luft und insbesondere um gefilterte Luft.

Bei einem weiteren bevorzugten Verfahren wird in dem Innenraum der Gehäuseeinrichtung wenigstens abschnittsweise und/oder wenigstens zeitweise ein Überdruck des gasförmigen Mediums erzeugt. Bevorzugt wird eine laminare Strömung verwendet. Besonders bevorzugt handelt es sich um einen geringen Überdruck, der bevorzugt bei weniger als 20%, bevorzugt bei weniger als 10% bezogen auf den Außendruck liegt. Auf diese Weise kann aus einzelnen Gehäuseteilen über Öffnungen die Luft wieder abgeleitet werden, sodass im Inneren der Gehäuseteile ein Luftstrom von gefilterter bzw. sauberer Luft vorherrscht.

Bevorzugt wird das gasförmige Medium daher gefiltert. Bei einem weiteren bevorzugten Verfahren werden zum Filtern des gasförmigen Mediums HEPA Filter eingesetzt.

Weitere Vorteile und Ausführungsformen ergeben sich aus den beigefügten Zeichnungen:

Darin zeigen:
- Fig. 1: Eine Gesamtdarstellung einer erfindungsgemäßen Vorrichtung 1;
- Fig. 2: Eine Detaildarstellung der in Figur 1 gezeigten Vorrichtung;
- Fig. 3: Eine Darstellung der Aufbewahrungseinrichtung einer erfindungsgemäßen Vorrichtung;
- Fig. 4: Eine teilweise Darstellung einer erfindungsgemäßen Vorrichtung;
- Fig. 5: Eine Detaildarstellung der in Figur 4 gezeigten Vorrichtung.

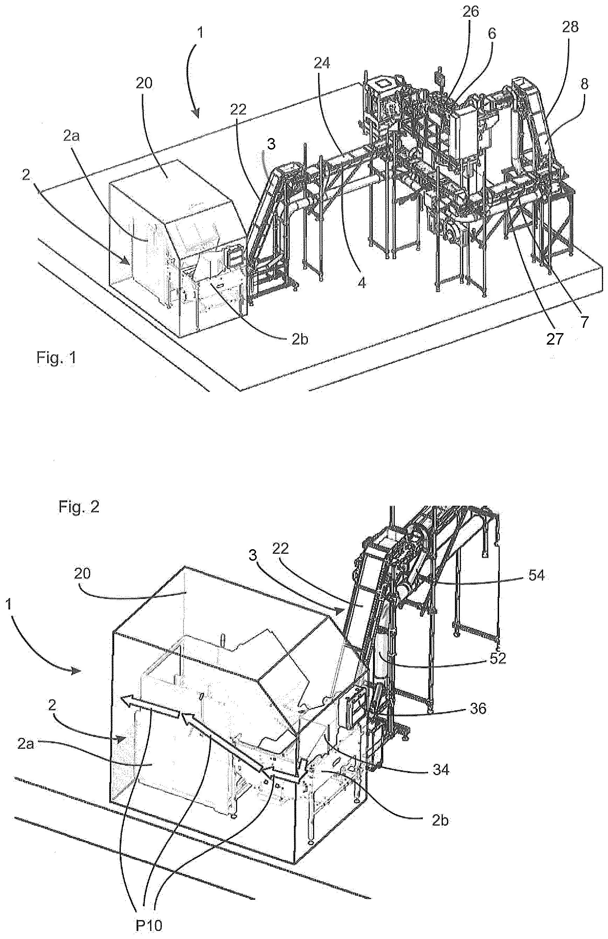

Figur 1 zeigt eine Gesamtdarstellung einer erfindungsgemäßen Vorrichtung. Hier ist das Gehäuse 20 der Aufbewahrungseinrichtung 2, hier genauer eines Kippers2a und eines Silos 2b gezeigt sowie die Gehäuse 22, 24, 25, 26, 27 und 28 der Transporteinrichtungen 3, 4, 5, 6, 7 und 8.. Die Gehäuseeinrichtung 24 umgibt hier ein Transportband 4. Die Gehäuseeinrichtung 22 umgibt einen Steilförderer, der die Kunststoffvorformlinge nach oben fördert und zwar in Richtung des besagten Transportbandes 4. Die Gehäuseeinrichtung 26 umgibt einen Rollensortierer 6. Der Rollensortierer 6 sortiert dabei die Vorformlinge nicht nur, sondern transportiert sie auch.

Daneben sind noch Gehäuseeinrichtungen 25 und 28 vorgesehen, welche die Transporteinrichtungen 5 und 8 umgeben, wobei es sich bei der Transporteinrichtung 8 um ein Zwischensilo handelt.

Figur 2 zeigt eine Teilansicht einer erfindungsgemäßen Vorrichtung 1 zum Behandeln und insbesondere Transportieren von Kunststoffvorformlingen. Dabei bezieht sich das Bezugszeichen 2 auf eine Aufbewahrungseinrichtung, in der eine Vielzahl von (nicht gezeigten) Kunststoffvorformlingen aufbewahrt wird. Dabei kann diese Aufbewahrungseinrichtung etwa einen Kipper 2a und ein Silo 2b aufweisen. Diese Kunststoffvorformlinge gelangen in das Silo 2b und können von dort mittels einer Transporteinrichtung 3, die hier als Steilförderer ausgebildet ist, transportiert werden. Das Bezugszeichen 20 kennzeichnet eine Einhausung bzw. ein Gehäuse, welches die Aufbewahrungseinrichtung 2 also hier insbesondere den Kipper 2a und das Silo 2b vollständig umgibt. Das Bezugszeichen 34 kennzeichnet eine Lüftereinrichtung, welche einen Luftstrom (Pfeile P10) innerhalb des Gehäuses 20 erzeugt. Um einen gerichteten Luftstrom zu erzeugen, weist das Gehäuse bevorzugt eine rückwärtige Öffnung auf, durch welche der Luftstrom austreten kann. Das Bezugszeichen 36 kennzeichnet eine Filtereinheit, über welche Umgebungsluft angesaugt wird und auf diese Weise auch gefiltert wird.

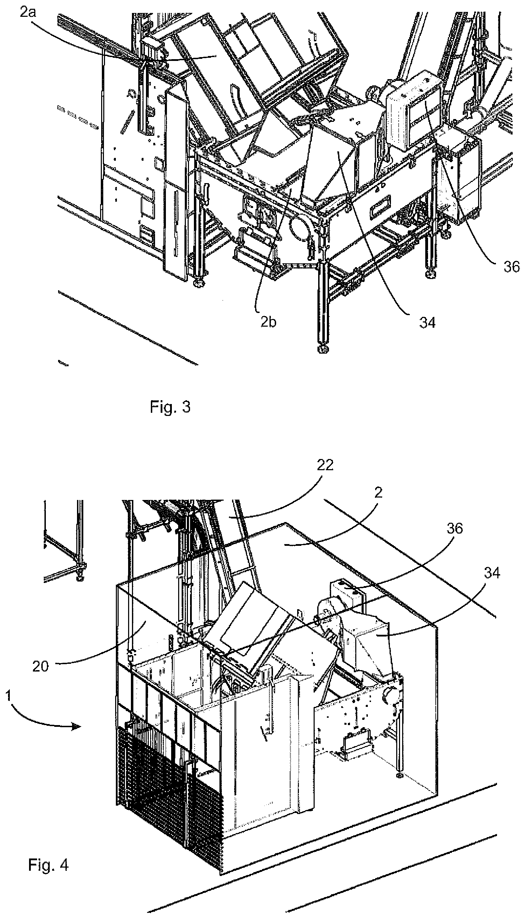

Figur 3 zeigt eine Detaildarstellung der in Figur 1 gezeigten Vorrichtung. Hier ist wieder die Lüftereinheit 34 dargestellt, sowie auch die Filtereinrichtung 36, welche zum Ansaugen von Luft geeignet ist. Das Bezugszeichen 2a kennzeichnet einen Kipper, der zum Zuführen der Kunststoffvorformlinge an das Silo 2b dient.

Figur 4 zeigt eine weitere Detaildarstellung der erfindungsgemäßen Vorrichtung. Man erkennt hier an der Rückseite des Gehäuses 20 eine Verschließvorrichtung, über welche eine Öffnung verschlossen werden kann, über welche dem Gehäuse 20 die Kunststoffvorformlinge zuführbar sind. Diese Verschließvorrichtung kann dabei gleichwohl Öffnungen zurücklassen, sodass auf diese Weise ein Luftstrom ausgehend von dem Lüfter 34 zu diesen Öffnungen erzeugt wird.

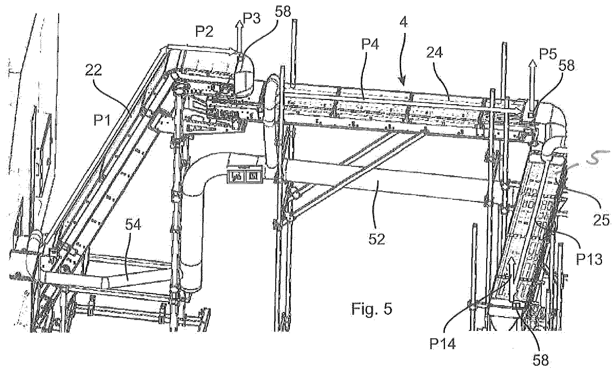

Figur 5 zeigt eine Detaildarstellung der in Figur 1 gezeigten Vorrichtung. Hierbei ist insbesondere wieder die Transporteinrichtung 4 erkennbar, welche von der Gehäuseeinrichtung 24 umgeben wird. Das Bezugszeichen 52 kennzeichnet eine Versorgungsleitung, welche zum Versorgen der Anlage mit Luft dient. Von dieser Versorgungsleitung 52 zweigen mehrere Abzweigleitungen 54 ab, die in bestimmte Bereiche der Vorrichtungen münden, wie beispielsweise in das Gehäuse 22 oder auch das Gehäuse 24. Die Pfeile P1, P2 und P3 zeigen den Verlauf der Strömung innerhalb der jeweiligen Gehäuse. Die Luftströmungen treten hier durch Öffnungen 58 wieder aus, sodass insgesamt hier die durch die Pfeile P1, P2 und P3 gezeigte Strömung erzeugt wird.

Ebenfalls werden innerhalb des Gehäuses 24 die Strömungen P4 und P5 erzeugt. Weitere Strömungen P13, P14 werden in einem Gehäuse 25 erzeugt. Auch hier ist wieder eine Öffnung 58 vorgesehen, über welche die Luft aus dem Gehäuse 25 austreten kann.

### Bezugszeichenliste

- 1: Vorrichtung
- 2: Aufbewahrungseinrichtung
- 2a: Kipper
- 2b: Silo
- 3: Transporteinrichtung
- 4: Transporteinrichtung
- 5: Transporteinrichtung
- 6: Transporteinrichtung
- 7: Transporteinrichtung
- 8: Transporteinrichtung
- 20: Einhausung bzw. Gehäuse
- 22: Gehäuseinrichtung
- 24: Gehäuseinrichtung
- 25: Gehäuse
- 26: Gehäuseinrichtung
- 28: Gehäuseinrichtung
- 34: Lüftereinrichtung
- 36: Filtereinheit
- 52: Versorgungsleitung
- 52: Rohrsystem
- 54: Abzweigleitungen
- 54: Rohrsystem
- 58: Öffnungen
- P1-P14: Luftstrom

## Patentansprüche

1. Vorrichtung (1) zum Behandeln von Kunststoffvorformlingen mit einer Aufbewahrungseinrichtung (2), welche zum Aufbewahren einer Vielzahl von Kunststoffvorformlingen dient und mit wenigstens einer Transporteinrichtung (3, 4, 5, 6, 7, 8), welche die Kunststoffvorformlinge entlang eines vorgegebenen Transportpfads von der Aufbewahrungseinrichtung (2) zu einer Verarbeitungseinrichtung (12) transportiert, **dadurch gekennzeichnet, dass** wenigstens ein Abschnitt der Aufbewahrungseinrichtung (2) und des Transportpfads von einer Gehäuseeinrichtung (20, 22, 24, 25, 26, 27, 28) eingehaust ist und eine Beaufschlagungseinrichtung (34, 52, 54) vorgesehen ist, welche einen Innenraum dieser Gehäuseeinrichtung (20, 22, 24, 25, 26, 27, 28) mit einem fließfähigen und insbesondere gasförmigen Medium beaufschlagt.

2. Vorrichtung (1) nach Anspruch 1,
**dadurch gekennzeichnet, dass**
die gesamte Aufbewahrungseinrichtung (2) und/oder der gesamte Transportpfad von einer oder mehreren Gehäuseeinrichtungen (20, 22, 24, 25, 26, 27, 28) eingehaust ist.

3. Vorrichtung (1) nach wenigstens einem der vorangegangenen Ansprüche,
**dadurch gekennzeichnet, dass**
die Vorrichtung entlang des Transportpfads eine Sortiereinrichtung (6) aufweist, welche dazu geeignet und bestimmt ist, die Kunststoffvorformlinge (10) zu sortieren.

4. Vorrichtung (1) nach Anspruch 3,
**dadurch gekennzeichnet, dass**
die Sortiereinrichtung zwei voneinander beabstandete drehbare Rollen aufweist, zwischen denen die Kunststoffvorformlinge (10) transportierbar sind.

5. Vorrichtung (1) nach wenigstens einem der vorangegangenen Ansprüche,
**dadurch gekennzeichnet, dass**
die Vorrichtung (1) eine Filtereinrichtung (36) zum Filtern des dem Innenraum der Gehäuseeinrichtung (20) zuzuführenden gasförmigen Mediums aufweist.

6. Vorrichtung (1) nach wenigstens einem der vorangegangenen Ansprüche,
**dadurch gekennzeichnet, dass**
die Beaufschlagungseinrichtung eine Lüftereinrichtung (34) zum Erzeugen eines Luftstroms aufweist.

7. Vorrichtung (1) nach Anspruch 6,
**dadurch gekennzeichnet, dass**
die Lüftereinrichtung (34) innerhalb einer Gehäuseeinrichtung (20) angeordnet ist, welche die Aufbewahrungseinrichtung (2) wenigstens teilweise umgibt.

8. Vorrichtung (1) nach wenigstens einem der vorangegangenen Ansprüche,
**dadurch gekennzeichnet, dass**
die Beaufschlagungseinrichtung (34, 52, 54) eine Versorgungsleitung (52) aufweist, welche dazu geeignet und bestimmt ist, einen von einer Lüftereinrichtung (34) erzeugten Gasstrom zu mehreren Positionen entlang des Transportpfads der Kunststoffvorformlinge zu verbringen.

9. Verfahren zum Behandeln von Kunststoffvorformlingen wobei in einer Aufbewahrungseinrichtung (2) eine Vielzahl von Kunststoffvorformlingen aufbewahrt wird und diese Kunststoffvorformlinge mit wenigstens einer Transporteinrichtung (4, 6, 8) entlang eines vorgegebenen Transportpfads von der Aufbewahrungseinrichtung (2) zu einer Verarbeitungseinrichtung (12) transportiert werden,
**dadurch gekennzeichnet, dass**
wenigstens ein Abschnitt der Aufbewahrungseinrichtung (2) und des Transportpfads (T) von einer Gehäuseeinrichtung (20, 22, 24, 25, 26, 27, 28) eingehaust ist und eine Beaufschlagungseinrichtung (34) einen Innenraum dieser Gehäuseeinrichtung (20, 22, 24, 25, 26, 27, 28) mit einem fließfähigen und insbesondere gasförmigen Medium beaufschlagt.

10. Verfahren nach Anspruch 9,
**dadurch gekennzeichnet, dass**
in dem Innenraum dieser Gehäuseeinrichtung wenigstens abschnittsweise und/oder wenigstens zeitweise ein Überdruck des gasförmigen Mediums erzeugt wird.

## Claims

1. Device (1) for handling plastic parisons with a storage device (2) which serves for storing a plurality of plastic parisons and with at least one transport device (3, 4, 5, 6, 7, 8) which transports the plastic parisons along a predetermined transport path from the storage device (2) to a processing device (12),
**characterised in that**
at least one portion of the storage device (2) and of the transport path is enclosed by a housing device (20, 22, 24, 25, 26, 27, 28) and an application device (34, 52, 54) is provided which applies a flowable and in particular gaseous medium to an interior of this housing device (20, 22, 24, 25, 26, 27, 28).

2. Device (1) according to claim 1,
**characterised in that**
the entire storage device (2) and/or the entire transport path is enclosed by one or more housing devices (20, 22, 24, 25, 26, 27, 28).

3. Device (1) according to at least one of the preceding claims,
**characterised in that**
the machine has a sorting device (6) along the transport path and is suitable and intended for sorting the plastic parisons (10).

4. Device (3) according to claim 3,
**characterised in that**
the sorting device has two rotatable rollers which are spaced apart from one another and between which the plastic parisons (10) can be transported.

5. Device (1) according to at least one of the preceding claims,
**characterised in that**
the machine (1) has a filter device (36) for filtering the gaseous medium to be fed to the interior of the housing device (20).

6. Device (1) according to at least one of the preceding claims,
**characterised in that**
the application device has a ventilator device (34) for generating an air stream.

7. Device (1) according to claim 6,
**characterised in that**
the ventilator device (34) is arranged inside a housing device (20) which at least partially surrounds the storage device (2).

8. Device (1) according to at least one of the preceding claims,
**characterised in that**
the application device (34, 52, 54) has a supply line (52) which is suitable and intended for bringing a gas stream generated by a ventilator device (34) to a plurality of positions along the transport path of the plastic parisons.

9. Method for handling plastic parisons, wherein a plurality of plastic parisons is stored in a storage device (2) and these plastic parisons are transported by at least one transport device (4, 6, 8) along a predetermined transport path from the storage device (2) to a processing device (12),
**characterised in that**
at least one portion of the storage device (2) and of the transport path (T) is enclosed by a housing device (20, 22, 24, 25, 26, 27, 28) and an application device (34) is provided which applies a flowable and in particular gaseous medium to an interior of this housing device (20, 22, 24, 25, 26, 27, 28).

10. Method according to claim 9,
**characterised in that**
a positive pressure of the gaseous medium is generated in the interior of this housing device at least in some portions and/or at least intermittently.

## Revendications

1. Dispositif (1) destiné à manipuler des préformes en plastique avec un équipement de conservation (2), lequel sert à conserver une pluralité de préformes en plastique et avec au moins un équipement de transport (3, 4, 5, 6, 7, 8), lequel transporte les préformes en plastique le long d'un chemin de transport prédéfini depuis l'équipement de conservation (2) vers un équipement d'usinage (12),
**caractérisé en ce que**
au moins une section de l'équipement de conservation (2) et du chemin de transport est logée par un équipement de logement (20, 22, 24, 25, 26, 27, 28) et un équipement de sollicitation (34, 52, 54) est prévu, lequel sollicite une espace intérieur de cet équipement de logement (20, 22, 24, 25, 26, 27, 28) avec un milieu apte à s'écouler et en particulier sous forme de gaz.

2. Dispositif (1) selon la revendication 1,
**caractérisé en ce que**
l'ensemble de l'équipement de conservation (2) et/ou l'ensemble du chemin de transport est logé par un ou plusieurs équipements de logement (20, 22, 24, 25, 26, 27, 28).

3. Dispositif (1) selon au moins l'une des revendications précédentes, **caractérisé en ce que**
le dispositif présente le long du chemin de transport un équipement de triage (6), lequel est conçu et déterminé pour trier les préformes en plastique (10).

4. Dispositif (1) selon la revendication 3,
**caractérisé en ce que**
l'équipement de triage présente deux rouleaux rotatifs espacés l'un de l'autre, entre lesquels les préformes en plastique (10) peuvent être transportées.

5. Dispositif (1) selon au moins l'une des revendications précédentes, **caractérisé en ce que**
le dispositif (1) présente un équipement de filtrage (36) pour filtrer le milieu sous forme de gaz à amener jusqu'à l'espace intérieur de l'équipement de logement (20).

6. Dispositif (1) selon au moins l'une des revendications précédentes, **caractérisé en ce que**
l'équipement de sollicitation présente un équipement de ventilateur (34) pour produire un écoulement d'air.

7. Dispositif (1) selon la revendication 6,
**caractérisé en ce que**
l'équipement de ventilateur (34) est agencé à l'intérieur d'un équipement de logement (20), lequel entoure au moins partiellement l'équipement de conservation (2).

8. Dispositif (1) selon au moins l'une des revendications précédentes, **caractérisé en ce que**
l'équipement de sollicitation (34, 52, 54) présente une conduite d'approvisionnement (52), laquelle est conçue et déterminée pour transférer un écoulement de gaz produit par un équipement de ventilateur (34) vers plusieurs positions le long du chemin de transport des préformes en plastique.

9. Procédé destiné à manipuler des préformes en plastique, dans lequel une pluralité de préformes en plastique est conservée dans un équipement de conservation (2) et ces préformes en plastique sont transportées avec au moins un équipement de transport (4, 6, 8) le long d'un chemin de transport prédéfini depuis l'équipement de conservation (2) vers un équipement d'usinage (12),
**caractérisé en ce que**
au moins une section de l'équipement de conservation (2) et du chemin de transport (T) est logée par un équipement de logement (20, 22, 24, 25, 26, 27, 28) et un équipement de sollicitation (34) sollicite une espace intérieur de cet équipement de logement (20, 22, 24, 25, 26, 27, 28) avec un milieu apte à s'écouler et en particulier sous forme de gaz.

10. Procédé selon la revendication 9,
**caractérisé en ce que**
une surpression du milieu sous forme de gaz est produite au moins par endroits et/ou au moins temporairement dans l'espace intérieur de cet équipement de logement.
